# EUROPEAN PATENT SPECIFICATION

(11) **EP 3 847 980 B1**
(45) Date of publication and mention of the grant of the patent: **24.07.2024**
(21) Application number: 21151225.6
(22) Date of filing: 12.01.2021
(51) Int. Cl.: A61B 17/221

(54) **EXTRACTOR CANNULA SECUREMENT AND PROCESSING**
BEFESTIGUNG UND VERARBEITUNG VON EXTRAKTIONSKANÜLEN
FIXATION ET TRAITEMENT DE CANULE DE SÉPARATEUR

(30) Priority: 13.01.2020 US 202062960348 P
(43) Date of publication of application: 14.07.2021
(73) Proprietor: Cook Medical Technologies LLC, Bloomington, IN 47404 (US)
(72) Inventor: BARTHOLOMAI, James Andrew, Louisville, KY Kentucky 40223 (US); JONES, Shannon, Greenwood, IN Indiana 46413 (US); SMITH, Johnny P, Worthington, IN Indiana 47471 (US); HUNDLEY, Lyle, Bloomington, IN Indiana 47403 (US); BUNCH, Kristen M, Bloomington, IN Indiana 47408 (US); ROSPORSKI, Carmen, Bloomington, IN Indiana 47401 (US)
(74) Representative: Warren, Caroline Elisabeth

(56) References cited:
- WO-A1-2009/121379
- DE-A1- 102012 222 356
- US-A- 5 064 428
- US-A1- 2009 030 427

## Description

This application claims priority to U.S. Provisional Application Ser. No. 62/960,348, filed January 13, 2020.

### BACKGROUND

Various organs and passages in the body are subject to the development of stones, calculi, and the like. For example, gallstones are a common problem in the United States and the most frequent cause of gallbladder inflammation. Calculi in other parts of the biliary system and/or the urinary system are also common places. Medical retrieval devices for capturing and extracting stones from various organs and passages are well known. Such devices typically comprise a retrieval basket attached to a distal end of an elongated member.

Urological baskets, and other baskets, often utilize a glue joint to secure the basket formation into the desired orientation. It is desired to use other methods to construct a stone management basket such as for the benefits of limiting the cost, both in labor and in material, to manufacture a stone management basket as well as for repeatability and other quality control factors.
US 5,064,428 describes a stone retrieval basket having superelastic metallic alloy wires attached to the distal end of an inner elongated member tube for retrieving calculi and crushing them against an outer introducer tube percutaneously inserted into a patient. The basket comprises kink-resistant superelastic metallic alloy wires such as nitinol forming a bulbous shape for capturing calculi therein. The ends of the superelastic wires of the basket are attached to the distal end of a inner elongated member tube with the aid of sleeves crimped thereon, which are soldered or spot welded in recesses about the distal end of the inner tube.
US 2009/0030427 describes a rigid device for use in percutaneous procedures to remove kidney stones directly from the kidneys. The rigid extractor uses a handle fixed to an outer rigid cannula, and an inner cannula to control an extraction device that may be removable from the inner cannula.
DE 10 2012 222 356 describes a medical catching-wire instrument, with a tube portion which can be inserted into a body duct of a patient and which comprises a tube sheath and a control element received therein so as to be axially movable relative thereto, and with a catching-wire element which is designed to be movable, through axial relative movement of tube sheath and control element, between a folded position, in which it is received in the tube sheath, and a functional position, in which it is deployed distally from the tube sheath. The metallic catching-wire element is metallic, and the tube sheath and the control element are non-metallic. A securing means fixes a proximal end area of the catching-wire element to a distal end area of the control element.
WO2009/121379 A1 describes a wire basket unit, comprising a balloon-shaped wire basket made of several wire strands, which extend between a front and a rear wire basket end and are formed by one or more wire pieces, and further comprising a fixing disk element, on which the wire strands are fixed at the front wire basket end while forming a front wire basket closure, the fixing disk element having at least one first and a second passage opening.

### BRIEF SUMMARY

Embodiments of the present disclosure relate to, among other things, securement and processing of medical retrieval devices. Each of the embodiments disclosed herein may include one or more of the features described in connection with any of the other disclosed embodiments.

Aspects of the invention are set out in the independent claim and preferred features are set out in the dependent claims. Preferred features of one aspect may be applied to other aspects.

An example medical retrieval device for retrieving stones, calculi, and the like, as well as polyps, foreign objects, and/or anatomical abnormalities from various organs and passages, is described but not claimed. The medical retrieval device comprises an elongated member, and a first wire and a second wire that forms a retrieval basket. The elongated member has a hollow passageway longitudinally positioned therebetween, and the first wire having first and second ends attached to the distal end of the elongated member and forming a first loop extending longitudinally and distally from said distal end of the elongated member. The second wire likewise has its first and second ends attached to the distal end of the elongated member, forming a second loop extending longitudinally and distally from the distal end of the elongated member. The second loop is interconnected distally to said first loop, which interconnection may include one or more of conjoined loops, cannula, weld, solder, or other connection means known in the art for the distal end of wire baskets. The elongated member and the first and second wires are secured by a crimp joint and a second joint, where such joints can be secured by a cannula, welding, and/or soldering in addition to and/or instead of crimping.

An aspect of the invention provides processing of the device disclosed above, processing comprising steps of placing all ends of the first wire and the second wire into an elongated member through a distal end of the elongated member, moving said elongated member to a position where the first loop and the second loop start their curvatures, placing said first and second wires and said elongated member through an alignment fixture such as a crimping or other securement fixture having thru cuts configured for the interlocked wires, aligning said elongated member in a desired position, and crimping said first and second wires and said elongated member, where such joints can be secured by a cannula, welding (including laser welding), glue, and/or soldering in addition to and/or instead of crimping.

Other systems, methods, features and advantages of the invention will be, or will become, apparent to one with skill in the art upon examination of the following figures and detailed description. The invention is defined by the appended claims.

### BRIEF DESCRIPTION OF THE DRAWINGS

**The** present disclosure can be better understood with reference to the following drawings and description. The components in the figures are not necessarily to scale, emphasis instead being placed upon illustrating the principles of the present disclosure. Moreover, in the figures, like-referenced numerals designate corresponding parts throughout the different views.
FIG. 1 is an illustration showing a distal portion of a retrieval device in accordance with certain non-limiting aspects of the present disclosure, where alternative basket and/or snare configurations may be used with the retrieval device.
FIG. 2 is an illustration showing an elongated member in accordance with certain aspects of the present disclosure.
FIG. 3 is an illustration showing a retrieval basket in accordance with certain aspects of the present disclosure.
FIGS. 4A-4D are illustrations showing various non-limiting crimp or other alignment fixture designs for processing of the device, where other securement means such as a cannula, welding (including laser welding), glue, and/or soldering in addition to and/or instead of crimping may be used.
FIG. 5 is an illustration showing how wires of the device may interact with, for example, a crimping fixture in accordance with certain aspects of the present disclosure.
FIG. 6 is an illustration showing assembly of the device in accordance with certain aspects of the present disclosure.
FIGS. 7A-7F are illustrations showing various cross sectional configurations, taken along line X-X of FIG. 6, near a crimp joint as described within the scope of the present disclosure.

### DETAILED DESCRIPTION

Various aspects are described below with reference to the drawings in which like elements generally are identified by like numerals. The relationship and functioning of the various elements of the aspects may better be understood by reference to the following detailed description. However, aspects are not limited to those illustrated in the drawings or explicitly described below. It also should be understood that the drawings are not necessarily to scale, and in certain instances details may have been omitted that are not necessary for an understanding of aspects disclosed herein, such as conventional material, construction, and assembly.

The terms "proximal" and "distal" are used herein to refer to the relative positions of the components of an exemplary medical device. When used herein, "proximal" refers to a position relatively closer to the exterior of the body or closer to a medical professional using the medical device. In contrast, "distal" refers to a position relatively further away from the medical professional using the medical device, or closer to the interior of the body.

In this specification the non-SI units 'inch' and 'lbs' are used, which may be converted to the Sl or metric unit according to the following conversion:
1 inch ≅ 25.4 mm
1 lbs ≅ 454 g

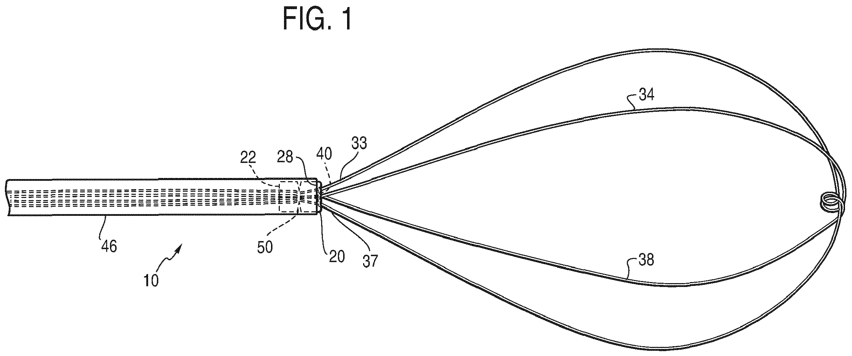

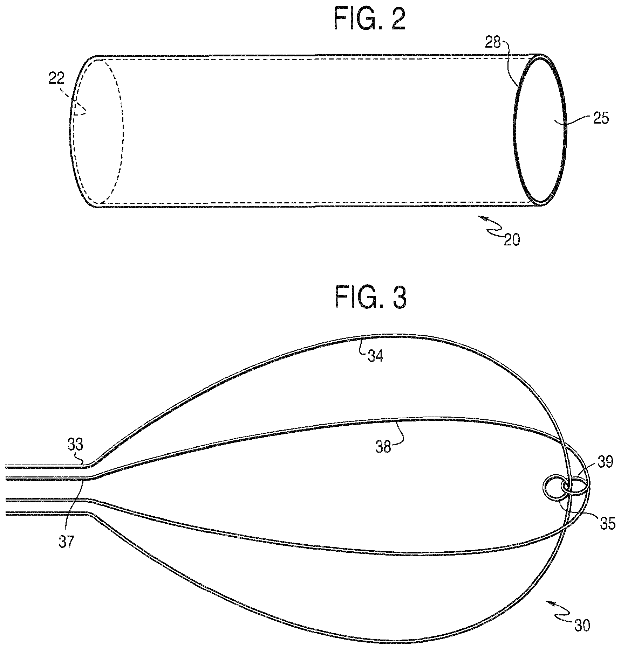

Referring to FIG. 1, a retrieval device 10 may comprise an elongated member 20 (such as, for example, a cannula), a retrieval basket 30, a crimp joint 50 and an additional/second joint 40. As illustrated by FIG. 2, the elongated member 20 may generally comprise a proximal end 22, a distal end 28, and a hollow passageway 25 longitudinally positioned therebetween. The elongated member 20 may be made of a metal such as stainless steel. Other flexible or rigid materials may also be utilized. The retrieval basket 30 may comprise a plurality of wires 34, 38 attached to the distal end 28 of the elongated member 20. The basket may include multiple loops of wires that interact with each other in order to be used in a stone management clinical setting, or for other retrieval uses when deployed a patient. The term "loop" should be understood to encompass a variety of shapes and not merely a circular or elliptical configuration, but also - by way of non-limiting example, helical, diamond-shaped, and/or other wire-shapes capable of defining a central volume to capture and/or carry a stone or other material. The basket can be preformed into any desired shape or size for capturing and removing calculi from the biliary or urinary system. Dimensions should be on scale with the size of the retrieval device. For example, the diameter of the device 10 may range from 1.5Fr to 4.5Fr. The wires 34, 38 may be made from a nickel titanium alloy such as nitinol. As illustrated by FIG. 3, in one embodiment, the basket 30 may comprise two wires 34, 38, each forming loops extending longitudinally and distally from the distal end of the elongated member 28. In some embodiments, the wires 34, 38 may be interconnected perpendicularly relative to each other at the distal end and/or along planes defined by the respective loop formed by each wire 34, 38. In one embodiment, the large loop of wire 38 includes a distal-end small loop 39 that interconnects/ links in a chain-like manner with a distal-end small loop 35 of the wire 34. Alternative embodiments may include different wire geometries (e.g. pie wire, round, square, D-shaped, etc.), wire quantities, and/or cannula geometries.

In one embodiment, the second joint 40 may be an adhesive such as glue. The adhesive may temporarily or permanently secure the wires 34, 38. Ideally, low viscosity cyanoacrylate glue, such as Loctite 4061, may be used for its strong adhesion to metal, wicking properties, and fast cure time. Alternative embodiments may include different glue compositions, and glue properties and application parameters should be selected to ensure appropriate adhesive dispersion. When using an adhesive, such as glue, as the second joint, a notchless cannula design may be advantageous to provide additional surface area for gluing and crimping.

In another embodiment, the second joint 40 may be a heat shrink. Ideally, a heat shrink, which may be activated using a heat gun, would have a melting temperature lower than the temperature at which the wires 34, 38 are formed (roughly 400°C) so that the wires 34, 38 are not adversely affected during the heat shrink process. Exemplary heat shrink materials may include PTFE and PEBAX. One advantage of this embodiment is that the wires 34, 38 are kept in a desired formation throughout the functioning life of the retrieval device 10. Another advantage of this embodiment is that it may replace the need to use a metal cannula and give the retrieval device 10 a higher degree of flexibility when used inside a scope for a procedure.

In one embodiment, processing of the retrieval device may generally include placing all four ends of the wires 34, 38 into the elongated member 20 through its hollow passageway 25, moving the elongated member 20 to the bottom of the wires 33, 37 where the wires 34, 38 end their curvature and go straight. Then, the wires 34, 38 and the elongated member 20 may be placed through an alignment fixture or other securement fixture such as crimping fixture 100 having thru cuts configured for the interlocked wires 34, 38 to ensure that the wires 34, 38 are aligned properly. Once the wires 34, 38 and the elongated member 20 are properly aligned, they may be crimped to form a crimp joint 50. A second joint 40 may be formed prior to forming the crimp joint 50.

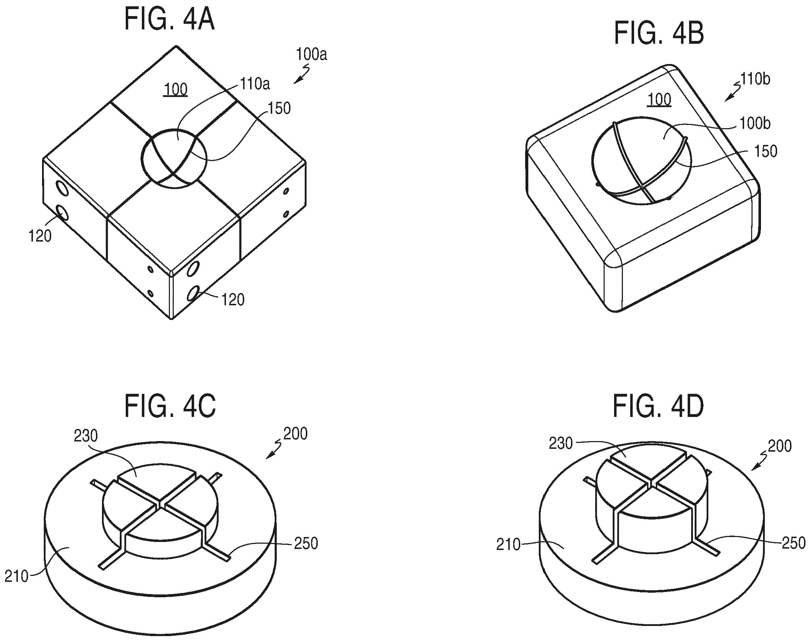

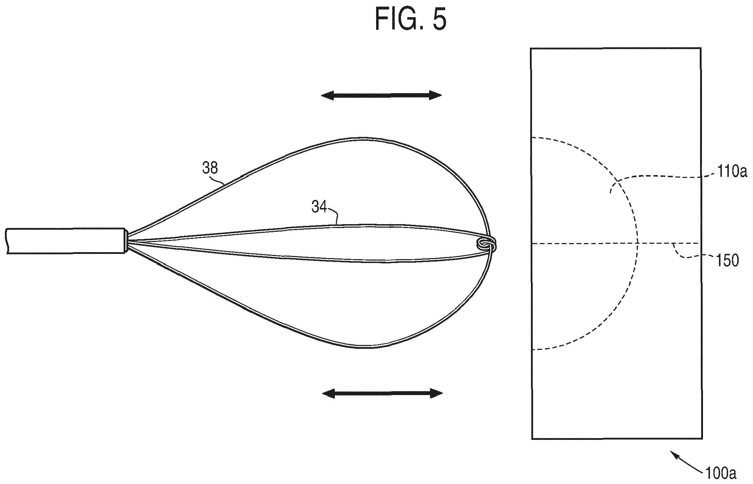

In one embodiment, formation of a crimp joint 50 may utilize a crimping fixture or other alignment fixture 100 to ensure that the wires 34, 38 hold their desired relationship during the crimping process. Specifically, crimping fixture or other alignment fixture 100a, 100b, 200 has a cross-hair thru cut 150, 250 all the way through the part to allow a distal or proximal end of the basket 30 to be fed through it before and/or after crimping. Alternatively, crimping fixture 100a, 100b, 200 may have a blind cut (not shown in figures) instead of thru cut 150, 200. Referring to FIGS. 4A-4D, crimping fixture 100a, 100b, 200 may have a thru cut 150, 250 that slides in the wires 34, 38 interconnected perpendicularly. Specifically, configurations illustrated in FIGS. 4A-4B have an indented area 110a, 110b in the middle to help the basket wires 34, 38 slide into the fixture. FIG. 5 illustrates how wires 34, 38 may interact with crimping fixture 100a. Indented area 110a may help wires 34, 38 to slide all the way into the fixture 100a through the thru cut 150. Other crimping fixtures as illustrated by FIG. 4B-4B may interact with wires 34, 38 in a similar manner. More specifically, the fixture of FIG. 4A has openings 120 on the side to allow assembly with screws (screws not shown in figures). The fixture of FIG. 4B, the fixture does not have openings 120 on the side for screw assembly, but instead has smaller area outside of the indented area 110b. The thru cut 150 should have a tight tolerance to keep the wires 34, 38 in line. For example, the length and width of thru cuts 150 may be 0.590 inches and 0.020 inches, respectively. As illustrated by FIGS. 4C and 4D, in another embodiment, crimping fixture 200 may comprise a base section 210 and an elongated section 230 to help get the fixture 200 closer to the crimping site. Specifically, the diameter of the base section 210 may be greater than the diameter of the elongated section 230. For example, the diameter of the base section 210 may be 0.750 inches while the diameter of the elongated section 250 may be 0.375 inches. Further, the thickness of base section 210 may be 0.157 inches. The thickness of the elongated section 250 may range from 0.079 inches to 0.154 inches. The crimping fixture 200 may also have the thru cut 250. As with alternative embodiment described above, the thru cut 250 should have a tight tolerance to keep the wires 34, 38 in line. Specific dimensions should be on scale with the size of the retrieval device 10, thus may vary.

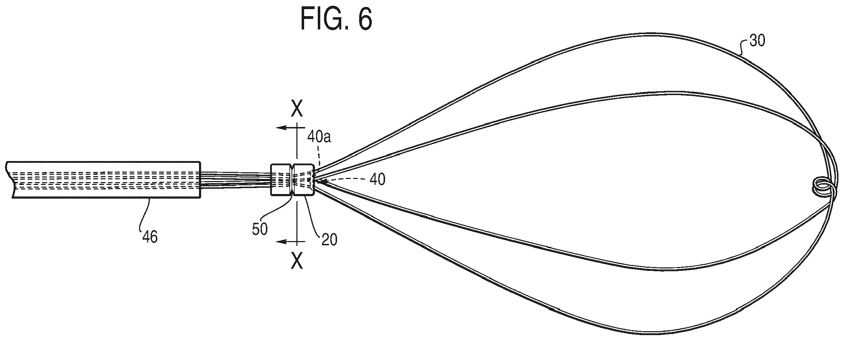

Referring to FIG. 6, processing of a device with a glue 40a or other secondary securement means (such as, by way of non-limiting example, weld or solder) 40a as part of a second joint 40 may include steps of aligning the wires 34, 38 inside an elongated member 20 to ensure that the wires 34, 38 are shaped correctly to form a basket of a desired configuration. Aligning may include pulling the wires 34, 38 into an alignment sheath (not shown) to assure a proper alignment and then removing the alignment sheath while maintaining the alignment. After removing the alignment sheath, the glue may be applied to the distal end 28 of the elongated member 20 or elsewhere within the elongated member 20. Once the glue 40a cures, a sheath 46 may be pulled over the elongated member 20 before or after the act of crimping. As shown in the drawings, the proximal half of the elongated member 20, where no glue 40a presents, may be crimped with the wires 34, 38. But, this crimp 50 and/or additional crimp joints 50 may be placed as needed or desired, proximal and/or distal of the glue or other secondary securement means in embodiments were such secondary securement means are present.

Processing an embodiment using a heat shrink as an elongated body 20 may comprise steps of placing all ends of the wires 34, 38 in a heat shrink, moving the heat shrink (20) to the wires' bottom portions 33, 37 where the wires 34, 38 end their curvature and go straight or substantially straight as compared to the loop portions. Further, processing may include turning a heat gun to a desired temperature, placing all ends of the wires 34, 38 and the heat shrink at a desired distance from the heat gun for a designated time. Once the heat shrink and the wires 34, 38 cool down and an inspection confirms a proper alignment, which may be perpendicular, one may proceed to further process the device 10.

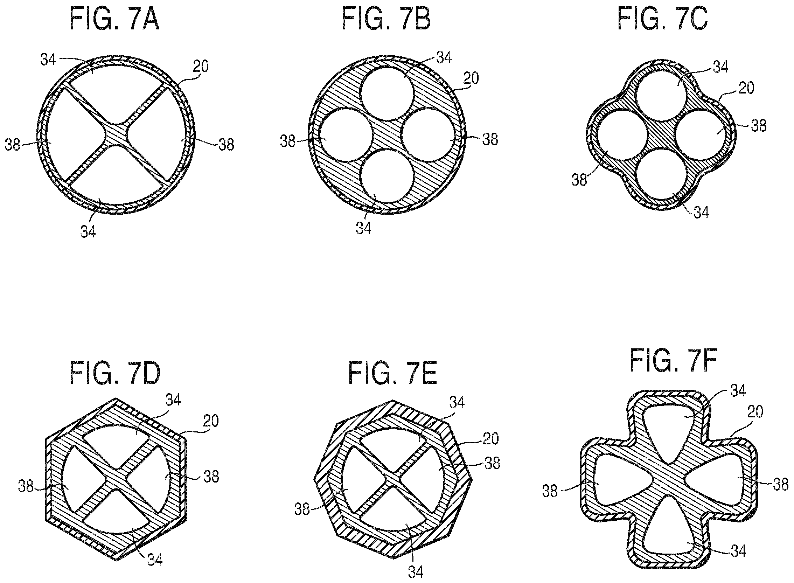

In another embodiment, a short crimper that can crimp down to 0.254mm (0.010") at a force greater than 45kg (100lbs). may be used to form a crimp joint 50. It is important to ensure that the pressure setting for the crimping fixture is set to the correct final outside diameter and that the pressure is high enough. The area of crimp joint 50 after crimping with a short crimper may have a hexagonally shaped cross-section, or another geometric shape (e.g., octagonal, circular, elliptical, or other shape, depending upon the shape of the crimping mechanism/surface contacting the crimp joint 50 during formation thereof, with reference to non-limiting examples in
FIGS. 7A-7F, where "geometric" is defined as "utilizing rectilinear or simple curvilinear motifs or outlines that bear little resemblance to natural/organic forms"). In some embodiments, as illustrated by FIGS. 7A-7F, the region near and/or including the crimp joint 50 may have different cross-sectional configurations (as viewed along line X-X of FIG. 6) after the wires are aligned and secured together. Certain embodiments, such as FIGS. 7A, 7D, 7E, and 7F, may advantageously include a pie-shaped wire, also called "delta wire", because the added angles may help keep the wires 34, 38 in the perpendicular relationship. These and/or any other crimping configurations and wire configurations or shapes may be used together with any of the aforementioned secondary means of securement, including by way of non-limiting example, glue, weld, and/or solder.

In summary, there is disclosed a medical retrieval device for retrieving stones, calculi, and the like from various organs and passages may comprising an elongated m em ber, and a first wire and a second wire that forms a retrieval basket. The wires forming the basket and the elongated member may be secured by a crim p joint and a second joint. Processing of the device may include placing all ends of the first wire and the second wire into an elongated member through a distal end of the elongated member, moving said elongated member to a position where the first loop and the second loop start their curvatures, placing said first and second wires and said elongated member through a crimping fixture having thru cuts configured for the interlocked wires, aligning said elongated member in a desired position, and crimping said first and second wires and said elongated member.

Although some preferred embodiments of the disclosure have been described, it should be understood that the disclosure is not so limited and modifications may be made without departing from the scope of the claims. The scope of the invention is defined by the appended claims which are to be interpreted in accordance with Article 69 EPC and its Protocol.

## Claims

1. A method of making a medical device (10) having a first wire (34), a second wire (38), and an elongated member (20), comprising the steps of:
placing all ends of the first wire (34) and the second wire (38) into an elongated member (20) through a distal end of the elongated member (20) so that the first wire (34) forms a first loop and the second wire (38) forms a second loop;
moving said elongated member (20) to a position where the first loop and the second loop start their curvatures;
placing said first and second wires (34, 38) and said elongated member (20) through an alignment fixture having thru cuts configured for the wires;
aligning said elongated member (20) in a desired position; and
crimping said first and second wires (34, 38) and said elongated member (20).

2. The method of claim 1, further comprising:
forming a first loop with a first wire (34);
forming a second loop with a second wire (38); and
distally interlocking the second wire (38) to said first loop prior to placing all ends of the first wire (34) and the second wire (38) into an elongated member (20) through a distal end of the elongated member (20).

3. The method of claim 2, further comprising configuring said first wire (34) and said second wire (38) perpendicularly.

4. The method of claim 1 or 2, wherein the alignment fixture further has a longitudinally elongated section that allows the fixture to get closer to a crimping site.

5. The method of any of claims 1 to 4, further comprising applying a second securement to the elongated member after placing all ends of the first wire (34) and the second wire (38) into said elongated member (20) through a distal end of said elongated member (20).

6. The method of any of claims 1 to 5, further comprising:
placing all ends of the first wire (34) and the second wire (38) into a heat shrink;
moving the heat shrink to a position where the first loop and the second loop start their curvatures;
heating the heat shrink and all ends of the wires at a desired distance for a designated time; and
cooling the heat shrink, the first wire (34), and the second wire (38) prior to placing all ends of the first wire (34) and the second wire (38) into said elongated member (20) through the distal end of said member (20).

7. The method of any preceding claim, wherein the crimping step includes using a crimper that crimps the elongated member in a geometric shape.

## Patentansprüche

1. Verfahren zum Fertigen einer medizinischen Vorrichtung (10), die einen ersten Draht (34), einen zweiten Draht (38) und ein längliches Element (20) aufweist, umfassend die Schritte:
Platzieren aller Enden des ersten Drahts (34) und des zweiten Drahts (38) in einem länglichen Element (20) durch ein distales Ende des länglichen Elements (20) hindurch, so dass der erste Draht (34) eine erste Schleife bildet und der zweite Draht (38) eine zweite Schleife bildet;
Bewegen des länglichen Elements (20) in eine Position, in der die erste Schleife und die zweite Schleife ihre Krümmungen beginnen;
Platzieren des ersten und des zweiten Drahts (34, 38) und des länglichen Elements (20) durch eine Ausrichtungshalterung hindurch, die durchgehende Schnitte aufweist, die für die Drähte konfiguriert sind;
Ausrichten des länglichen Elements (20) in einer gewünschten Position; und
Crimpen des ersten und des zweiten Drahts (34, 38) und des länglichen Elements (20).

2. Verfahren nach Anspruch 1, des Weiteren umfassend:
Bilden einer ersten Schleife mit einem ersten Draht (34) ;
Bilden einer zweiten Schleife mit einem zweiten Draht (38); und
distales Ineinandergreifen des zweiten Drahts (38) mit der ersten Schleife vor dem Platzieren aller Enden des ersten Drahts (34) und des zweiten Drahts (38) in einem länglichen Element (20) durch ein distales Ende des länglichen Elements (20) hindurch.

3. Verfahren nach Anspruch 2, des Weiteren umfassend Konfigurieren des ersten Drahts (34) und des zweiten Drahts (38) rechtwinklig zueinander.

4. Verfahren nach Anspruch 1 oder 2, wobei die Ausrichtungshalterung des Weiteren ein in Längsrichtung verlängertes Segment aufweist, das ermöglicht, dass die Halterung näher an eine Crimpstelle heran kommt.

5. Verfahren nach einem der Ansprüche 1 bis 4, des Weiteren umfassend Anwenden einer zweiten Sicherung auf das längliche Element nach dem Platzieren aller Enden des ersten Drahts (34) und des zweiten Drahts (38) in dem länglichen Element (20) durch ein distales Ende des länglichen Elements (20) hindurch.

6. Verfahren nach einem der Ansprüche 1 bis 5, des Weiteren umfassend:
Platzieren aller Enden des ersten Drahts (34) und des zweiten Drahts (38) in einem Wärmeschrumpfschlauch;
Bewegen des Wärmeschrumpfschlauchs in eine Position, in der die erste Schleife und die zweite Schleife ihre Krümmungen beginnen;
Erwärmen des Wärmeschrumpfschlauchs und aller Enden der Drähte in einem gewünschten Abstand für eine vorgesehene Zeit; und
Kühlen des Wärmeschrumpfschlauchs, des ersten Drahts (34) und des zweiten Drahts (38) vor dem Platzieren aller Enden des ersten Drahts (34) und des zweiten Drahts (38) in dem länglichen Element (20) durch das distale Ende des Elements (20) hindurch.

7. Verfahren nach einem der vorhergehenden Ansprüche, wobei der Crimpschritt Verwenden einer Crimpzange einschließt, welches das längliche Element zu einer geometrischen Form crimpt.

## Revendications

1. Procédé de fabrication d'un dispositif médical (10) comportant un premier fil (34), un second fil (38), et un élément allongé (20), comprenant les étapes suivantes :
placer toutes les extrémités du premier fil (34) et du second fil (38) dans un élément allongé (20) à travers une extrémité distale de l'élément allongé (20) de manière à ce que le premier fil (34) forme une première boucle et que le second fil (38) forme une seconde boucle ;
déplacer ledit élément allongé (20) jusqu'à une position où la première boucle et la seconde boucle commencent leurs courbures ;
placer lesdits premier et second fils (34, 38) et ledit élément allongé (20) à travers un dispositif d'alignement ayant des coupes transversales configurées pour les fils ;
aligner ledit élément allongé (20) dans une position souhaitée ; et
sertir lesdits premier et second fils (34, 38) et ledit élément allongé (20).

2. Procédé selon la revendication 1, comprenant en outre :
la formation d'une première boucle avec un premier fil (34) ;
la formation d'une seconde boucle avec un second fil (38) ; et
l'emboîtement distal du second fil (38) à ladite première boucle avant de placer toutes les extrémités du premier fil (34) et du second fil (38) dans un élément allongé (20) à travers une extrémité distale de l'élément allongé (20).

3. Procédé selon la revendication 2, comprenant en outre la configuration dudit premier fil (34) et dudit second fil (38) perpendiculairement.

4. Procédé selon la revendication 1 ou 2, la fixation d'alignement ayant en outre une section allongée longitudinalement qui permet à la fixation de se rapprocher d'un site de sertissage.

5. Procédé selon l'une quelconque des revendications 1 à 4, comprenant en outre l'application d'une seconde fixation à l'élément allongé après avoir placé toutes les extrémités du premier fil (34) et du second fil (38) dans ledit élément allongé (20) à travers une extrémité distale dudit élément allongé (20).

6. Procédé selon l'une quelconque des revendications 1 à 5, comprenant en outre :
le placement de toutes les extrémités du premier fil (34) et du second fil (38) dans une gaine thermorétractable ;
le placement de la gaine thermorétractable jusqu'à une position où la première boucle et la seconde boucle commencent leurs courbures ;
le chauffage de la gaine thermorétractable et de toutes les extrémités des fils à une distance souhaitée pendant une durée déterminée ; et
le refroidissement de la gaine thermorétractable, du premier fil (34) et du second fil (38) avant de placer toutes les extrémités du premier fil (34) et du second fil (38) dans ledit élément allongé (20) à travers l'extrémité distale dudit élément (20).

7. Procédé selon n'importe quelle revendication précédente, l'étape de sertissage comprenant l'utilisation d'une sertisseuse qui sertit l'élément allongé selon une forme géométrique.
